# EUROPEAN PATENT SPECIFICATION

(11) **EP 2 384 148 B1**
(45) Date of publication and mention of the grant of the patent: **14.01.2015**
(21) Application number: 09764684.8
(22) Date of filing: 03.12.2009
(51) Int. Cl.: A61B 17/04

(54) **FIXTURE FOR MAINTAINING THE TENSION APPLIED TO A SUTURE DURING INTRACORPOREAL PROCEDURES**
ARMATUR ZUR AUFRECHTERHALTUNG DER AN EIN NAHTMATERIAL ANGELEGTEN SPANNUNG WÄHREND INTRAKORPORALER EINGRIFFE
FIXATION POUR MAINTENIR LA TENSION APPLIQUÉE À UNE SUTURE AU COURS DE PROCÉDURES INTRACORPORELLES

(30) Priority: 05.12.2008 US 120193 P
(43) Date of publication of application: 09.11.2011
(73) Proprietor: Cook Medical Technologies LLC, Bloomington, IN 47404 (US)
(72) Inventor: MCLAWHORN, Tyler, E., Winston-salem NC 27106 (US)
(74) Representative: Wächter, Jochen
(86) International application number: PCT/US2009/066564
(87) International publication number: WO 2010/065727

(56) References cited:
- EP-A1- 1 884 199
- EP-A1- 1 987 779
- EP-A2- 1 844 715
- EP-A2- 1 964 521
- WO-A1-03/051204

## Description

### FIELD

The present invention relates generally to devices, systems, and methods for sustaining the tension applied to sutures that are being fixed to a material (e.g., bodily tissue, fabrics, cloth, polymers, elastomers, plastics, and rubber), such as for closing perforations in the material.

### BACKGROUND

Openings or perforations in the walls of internal organs and vessels may be naturally occurring, or formed intentionally or unintentionally. These openings may be used to gain access to adjacent structures of the body, such techniques being commonly referred to as transluminal procedures. For example, culdoscopy was developed over 70 years ago, and involves transvaginally accessing the peritoneal cavity by forming an opening in the *cul de sac.* This access to the peritoneal cavity allows medical professionals to visually inspect numerous anatomical structures, as well as perform various procedures such as biopsies or other operations, such as tubal ligation. Many transluminal procedures for gaining access to various body cavities using other bodily lumens have also been developed. Natural orifices such as the mouth, nose, ear, anus or vagina may provide access to such bodily lumens and cavities. The bodily lumen(s) of the gastrointestinal tract are often endoscopically explored and can be utilized to provide access to the peritoneal cavity and other body cavities, all in a minimally invasive manner. U.S. Patent Publication No. 2008/0132948A1 published June 5, 2008, discloses an example of such a procedure.

In order to permanently close naturally occurring, as well as intentionally or unintentionally formed perforations, and allow the tissue to properly heal, numerous medical devices and methods have been developed that employ sutures, adhesives, clips, tissue anchors and the like. Where sutures are used, e.g., with or without anchoring devices, the sutures must be tied or otherwise connected together.

Manually tying sutures strands together to close a perforation also can be very complex and time consuming. For example, a significant level of skill and coordination is required by the medical professional, especially when the perforation and sutures are difficult to access within the body, such as in endoscopic or laparoscopic procedures. The numerous difficulties with manually tying sutures are well documented. Likewise, many other endoscopic and laparoscopic procedures, as well as other applications involving a material (e.g., fabrics, cloth, polymers, elastomers, plastics and rubber) located in a difficult to access location, may require the tensioning and/or tying of sutures and suffer from similar difficulties.

A suture tensioning device for engaging and pulling a length of suture through a knotting element is disclosed in EP 1 844 715 A2 which is considered the closest prior art document. The device comprises an elongated shaft or sheath, a grasping element and a knotting element attached to the shaft at the distal end. The suture grasping element engages the suture and pulls it through the knotting element to tension the suture. The suture can be locked and unlocked in the knotting element by actuating a locking mechanism.

Another suture tensioning device is disclosed in EP 1 987 779 A1. The device comprises a shaft with a wheel mounted thereon. The wheel is provided with a forked pin which engages the suture. The suture is tensioned by turning the wheel.

Another device for tensioning a suture is disclosed in WO 03/051204. The device comprises a sleeve shaped member, a sliding element disposed therein and a holding element. The suture extends through the sliding element and is fixedly attached to the holding element with one end of the suture. The other end of the suture forms a moveable knot. The suture can be tensioned by moving the sliding element towards the knot while holding back the holding element.

### BRIEF SUMMARY

The present invention provides a tension sustaining fixture, a medical system, and related methods for suturing a material (e.g., tissue) and for tying sutures to close a perforation made in the material (i.e., during intracorporeal procedures). One embodiment of a tension sustaining fixture, constructed in accordance with the teachings of the present invention, generally comprises a main body having a first passageway, the main body capable of being coupled along the longitudinal axis of the suturing device. A side retaining sleeve is unitarily formed with or coupled to the main body and has an internal surface defining a second passageway intersecting with the first passageway. A closure mechanism engages the side retaining sleeve. The closure mechanism further defines the second passageway and sized to slidably receive at least one suture having a proximal end and a distal end. The first passageway and the second passageway of the tension sustaining fixture intersect at an angle (α) that preferably has a magnitude less than about 90 degrees, and more preferably less than about 45 degrees.

The distal end of a suture follows a path from the first passageway through the second passageway and the closure mechanism. The closure mechanism is operable between a locked configuration and an unlocked configuration, whereby, the tension applied to the suture can be maintained when the closure mechanism is in its locked configuration.

The amount of friction applied to a suture is negligible when the closure mechanism is in its unlocked configuration. The closure mechanism may be a rotatable member, such as a stopcock, positioned to close the passage way or a pin vise collet having a locking cap. Minute adjustments to the amount of friction applied to the sutures is accomplished by either turning the stopcock partially open or by loosening the locking cap on the pin vise collet, and the tension on the sutures may be manually adjusted.

According to one aspect of the present invention, the intracorporeal procedure may be an endoscopic or laparoscopic procedure using an endoscope and wherein the elongated tube or sheath of the tension sustaining fixture is sized to be inserted through a working channel of the endoscope.

According to another aspect of the present invention, a medical system useful for suturing tissue in a patient during an intracorporeal procedure comprises the tension sustaining fixture according to the present invention. The medical system further comprises a suturing device having a handle and an outer sheath that slidably receives the distal end of the sutures for delivery to the tissue within the patient. The tension sustaining fixture having a main body is located between the handle and the outer sheath of the suturing device. The side retaining sleeve intersects with the main body at an angle (α). The closure mechanism is coupled to the side retaining sleeve. At least one suture has a proximal end that is fed from the outer sheath through the tension sustaining fixture to be external to the patient. The closure mechanism is operable between a locked configuration and an unlocked configuration with the amount of tension applied to the sutures being maintained when the closure mechanism is in its locked configuration.

An exemplary method for closing perforations made in a material by means of the tension sustaining fixture according to the present invention comprises the steps of placing the distal end of the suturing device at a location that is proximate to the material; making a perforation in the material; securing the distal end of at least one suture strand proximate to the perforation made in the material; providing appropriate tension to the suture(s); sustaining the applied tension using a tension sustaining fixture; and securing the sutures to close the perforation.

In this method the main body of the tension sustaining fixture is coupled to the suturing device, the side retaining sleeve intersects with the main body at an angle (α), and the closure mechanism is coupled to the side retaining sleeve. The proximal end of at least one suture is fed from the perforation in the material through the side retaining sleeve and closure mechanism of the tension sustaining fixture. The appropriate tension is provided and manipulated manually or through the use of an instrument or robot. The applied tension is sustained by closing the closure mechanism of the tension sustaining fixture. The closure mechanism may be either a rotatable member, such as a stopcock, or a pin vise collet having a locking cap. The tension to the sutures may be adjusted as necessary to effectively close the perforation by opening the closure mechanism and manually adjusting the tension; or by partially opening or closing the closure mechanism when the closure mechanism is a rotatable member.

Further areas of applicability will become apparent from the description provided herein. It should be understood that the description and specific examples are intended for purposes of illustration only and are not intended to limit the scope of the present disclosure.

### BRIEF DESCRIPTION OF THE DRAWINGS

The drawings described herein are for illustration purposes only and are not intended to limit the scope of the present disclosure in any way.
- **Fig. 1**: is a perspective view of a system according to one embodiment of the present invention for providing tension to the sutures used to close perforations in the walls of organs and vessels in a patient during an endoscopic procedure;
- **Fig. 2A**: is a perspective view of the tension sustaining fixture according to one embodiment of the present invention showing a closure mechanism in its fully open condition;
- **Fig. 2B**: is a perspective view of the tension sustaining fixture of Figure 2A showing the closure mechanism in a partially closed condition;
- **Fig. 2C**: is a perspective view of a tension sustaining fixture of Figure 2A showing the closure mechanism in its fully closed position;
- **Fig. 3A**: is a cross-sectional view of the closure mechanism of Figure 2A in its fully open position taken along line B-B as defined in Figure 2C;
- **Fig. 3B**: is a cross-sectional view of the intersection between the first passage way and the second passage way of the tension sustaining fixture according to one embodiment of the present invention;
- **Fig. 4A**: is a perspective view of a tension sustaining fixture according to another embodiment of the present invention;
- **Fig. 4B**: is a perspective view of the tension sustaining fixture of Figure 4A with a locking cap; and
- **Fig. 5**: is a schematic representation of an exemplary method for sustaining the tension applied to suture strands connected to a material.

### DETAILED DESCRIPTION

The following description is merely exemplary in nature and is in no way intended to limit the present invention or its application or uses. It should be understood that throughout the description and drawings, corresponding reference numerals indicate like or corresponding parts and features. In the present specification, the term "proximal" refers to a direction that is generally towards a physician during a medical procedure, while the term "distal" refers to a direction that is generally towards a target site within a patient's anatomy during a medical procedure.

It will be recognized by those skilled in the art that, while the methods described in this disclosure generally include placing the tissue devices in tissue through an internal bodily lumen, it will be recognized that these systems, devices and methods may be used on any layer of material (e.g. fabrics, cloth, polymers, elastomers, plastics and rubber) that may or may not be associated with a human or animal body and a bodily lumen. For example, the systems, devices and methods can find use in laboratory and industrial settings for placing devices through one or more layers of material that may or may not find application to the human or animal body, and likewise closing holes or perforations in layers of material that are not bodily tissue.

One embodiment of the present invention, constructed in accordance with the teachings herein, generally provides a tension sustaining fixture for use with a suturing device that maintains the tension applied to sutures being fixed to a material, such as in closing perforations in the material. Although, more broadly applicable, the following description of the tension sustaining fixture, device and method of utilizing such device in the present disclosure employs an endoscopic or laparoscopic procedure as a convenient means through which the device and method can be adequately depicted and described.

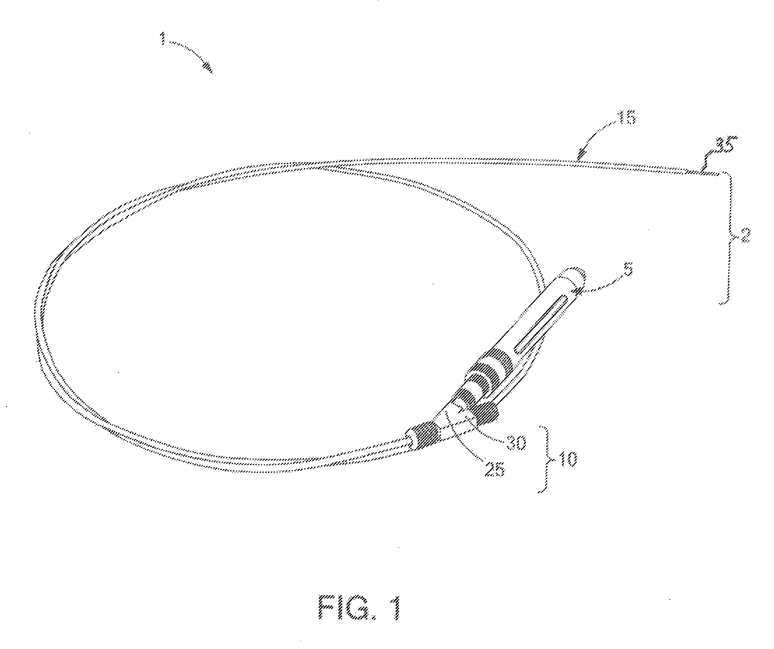

Referring to Figure 1, the medical system 1 comprises a suturing device 2 and a tension sustaining fixture 10. The suturing device 2 may have a handle 5 and an outer sheath 15. The suturing device may be any type of a device known in the art including those that apply T-anchors, staples, tacks, clips or the like. According to one aspect of the present invention, the intracorporeal procedure may be an endoscopic or laparoscopic procedure using an endoscope where the suturing device 2 and the tension sustaining fixture 10 are integrated together such that the elongated tube or sheath 15 is shared by and is considered to be part of both the suturing device 2 and the tension sustaining fixture 10. The elongated tube or sheath 15 is sized to be inserted through a working channel of the endoscope.

One example of a suturing device 2 is one that utilizes a set of tissue anchors having an anchoring member attached to one or more sutures 35. Examples of such exemplary devices are disclosed in U.S. Patent Publication No. 2008/0132948A1, and exemplary staples are disclosed U.S. Patent Publication No. 2009/0048613A1 published on February 19, 2009, and exemplary tacks are disclosed in U.S. Patent Publication No. 2009/0270912A1 published on October 29, 2009. These tissue anchors are loaded into a delivery needle, and here the suture(s) are fed through the outer sheath 15 of the suturing device 2. The crossbar/anchoring member of the tissue anchor is passed through the bodily wall adjacent to the periphery of the perforation in the wall. Thus each tissue anchor ends up being on the distal side of the bodily wall with the opposite ends of the suture 35 remaining on the proximal side of the bodily wall. The sutures 35 are then tensioned to reduce the distance between the tissue anchors and to compress the bodily wall proximate to the perforation. Finally, the ends of the sutures 35 are fixed together in order to maintain the compression of the bodily wall and to close the perforation.

The ends of the sutures 35 may be manually tied or a suture lock may be employed (see, e.g., U.S. Patent Publication No. 2008/0300629A1 published on December 4, 2008 and U.S. Patent Publication No. 2009/0069847A1 published on March 12, 2009). Although this example describes the use of T-anchors, one skilled in the art will recognize that other types of suturing devices and methods may be utilized without departing from the scope of the present invention, including staples (see, e.g., U.S. Patent Publication No. 2009/0048613A1), tacks (see, e.g., U.S. Patent Publication No. 2009/0270912A1), clips, and similar devices.

Sutures 35 are generally placed in tension by the natural elasticity of the bodily tissue pulling the sutures 35 distally. At the same time the sutures 35 may be pulled in the proximal direction to further apply and adjust suture tension, lock suture position, orient the suturing device, and to further close the perforation. This tension may be applied and manipulated by hand, using a device or instrument, or by a robot.

The tension sustaining fixture 10 is located at the proximal end of the system 1, and preferably between the handle 5 and the outer sheath 15 of the suturing device 2. The tension sustaining fixture 10 may be unitarily formed with, integrally mounted on, or fastened to the suturing device 2.

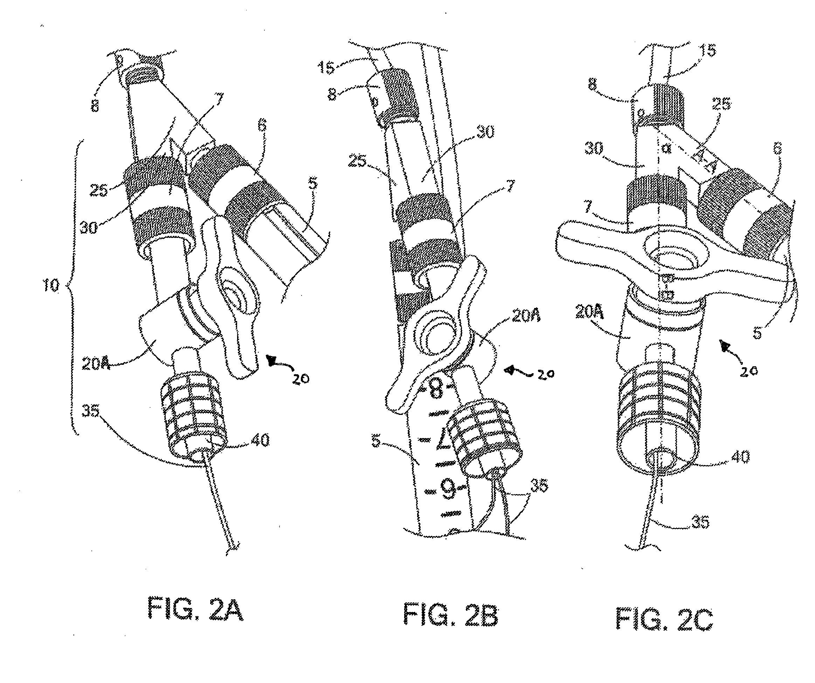

Referring now to Figures 2A-2C, 3A, 3B, and 4A, the tension sustaining fixture 10 comprises a main body 25 having a first passageway 31, the main body 25 capable of being in communication with the elongated lumen or sheath along the longitudinal axis A-A of the suturing device 5. A side retaining sleeve 30 is integrally formed with or coupled to the main body 25 and has an internal surface that defines a second passageway 32 intersecting with the first passageway 31. A closure mechanism 20 engages the side retaining sleeve 30. The closure mechanism 20 defines a passageway 40 extending as part of the second passageway 32 and is sized to slidably receive at least one suture 35. The suture 35 used in the suturing device 2 has a proximal and distal end. The distal end of a suture 35 extends through a catheter or needle 11 near the area in the lumen to be sutured. The proximal end of a suture 35 is extended from the first passageway 31 through the second passageway 32/40 of the tension sustaining fixture 10.

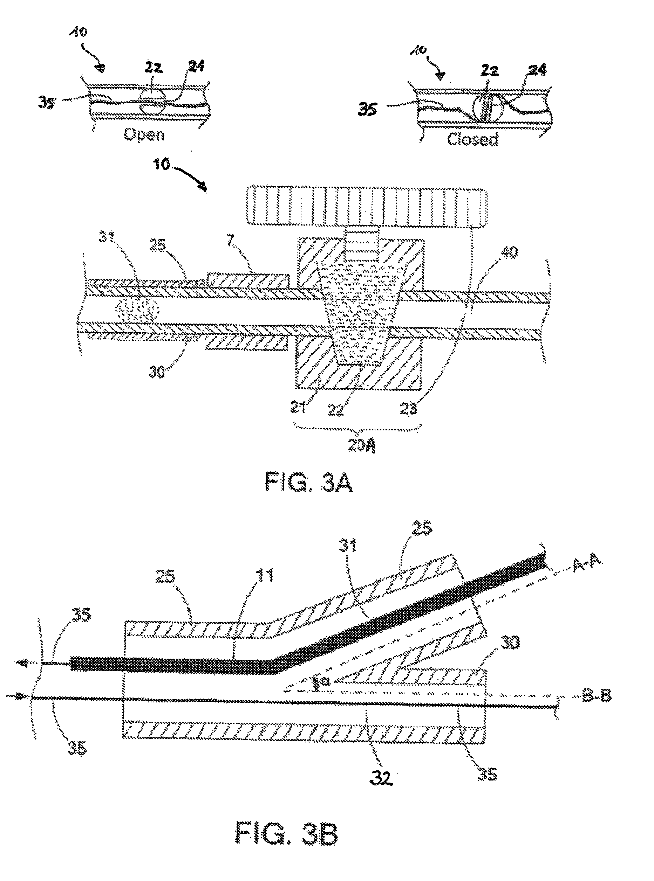

As also shown in Figures 2A-2C and Figure 3A, the main body 25 of the tension sustaining fixture 10 and the handle 5 of the suturing device 2 are in-line with each other along axis A-A. The side retaining sleeve 30 and closure mechanism 20 of the tension sustaining fixture 10 are in-line with each other along axis B-B. The first passageway of the main body 25 positioned along axis A-A and the second passageway 40 of the side retaining sleeve 30 are positioned along axis B-B and intersect with each other making an angle (α) as shown in Figures 2C and 3B. This angle (α) is less than about 90 degrees, with less than about 45 degrees being preferred.

The main body 25 of the tension sustaining fixture 10 may be integrally formed with or coupled to both the handle 5 and outer sleeve 15 of the suturing device 2. The retaining sleeve 30 and the main body 25 are preferably unitarily formed. The retaining sleeve 30 may be unitarily formed with or integrally coupled to the closure mechanism 20. The coupling of the main body 25 of the tension sustaining fixture 10 to the handle 5 and the outer sleeve 15 of the suturing device 2 and the coupling of the retaining sleeve 30 to the closure mechanism 20 of the tension sustaining fixture 10 can be accomplished using any type of fastener (see 6, 7, & 8, respectively) known to one skilled in the art, such as threaded connectors, nuts, or quick connect fittings, among others.

The closure mechanism is operable between a locked configuration (Figure 2C) and an unlocked configuration (Figure 2A). The amount of tension applied to the sutures 35 is maintained when the closure mechanism is in a locked configuration (Figure 2C) or a partially locked configuration (i.e. between the unlocked and fully locked configurations). The amount of friction placed on the sutures 35 by the closure mechanism 20 is negligible when the closure mechanism is in an unlocked configuration. Once tension is applied to the suture 35 and is desired to be maintained, the closure mechanism 20 can be closed in order for the tension to be maintained.

In one embodiment of the present invention, the closure mechanism 20 may be a rotatable member 20A positioned to close the passage way, such as a stopcock as shown in Figures 2A-2C. A stopcock 20A may be comprised of a rotatable key 22, a fixed body 21, and a handle 23. The handle 23 is coupled to the key 22 is used to rotate the key 22 within the confines of the body 21 from its fully open position to its fully closed position (i.e. unlocked and locked configurations). It is also possible to partially rotate the key 22 to a position between the fully open and closed positions. The suture 35 is fed through the opening (indicated by dotted lines) in the key 22 of the stopcock. When the stopcock 20A is closed, the suture becomes "pinched" or otherwise frictionally engaged between the body 21 and the key 22, thereby maintaining the tension on the suture. Opening the stopcock 20A either partially or fully allows the suture to slide through the opening in the key 22 and hence through the second passageway 32/40, and allows the user to manually adjust the tension on the sutures until the appropriate tissue approximation is obtained.

The applied tension can then be maintained by closing the stopcock 20A as shown in Figure 2C. Once the stopcock 20A is closed, the tension is sustained, and the user's hands are free to operate the suturing device 2 in order to provide a permanent lock on the suture. The tension on the sutures can be released by simply opening the stopcock 20A as shown in Figure 2A. In the open position, adjustments can be made to the applied tension, which then can be sustained by closing the stopcock 20A. In this embodiment, minute adjustments in the amount of friction applied to the sutures 35 can be made by slightly turning the stopcock 20A from its closed configuration in the direction of its open configuration as shown in Figure 2B. The user may provide a slight pulling action on the sutures at this time to achieve the desired level of tension and tissue approximation. The stopcock 20A is also capable of engaging the suture 35 such that tension on the suture 35 may be increased and then automatically maintained.

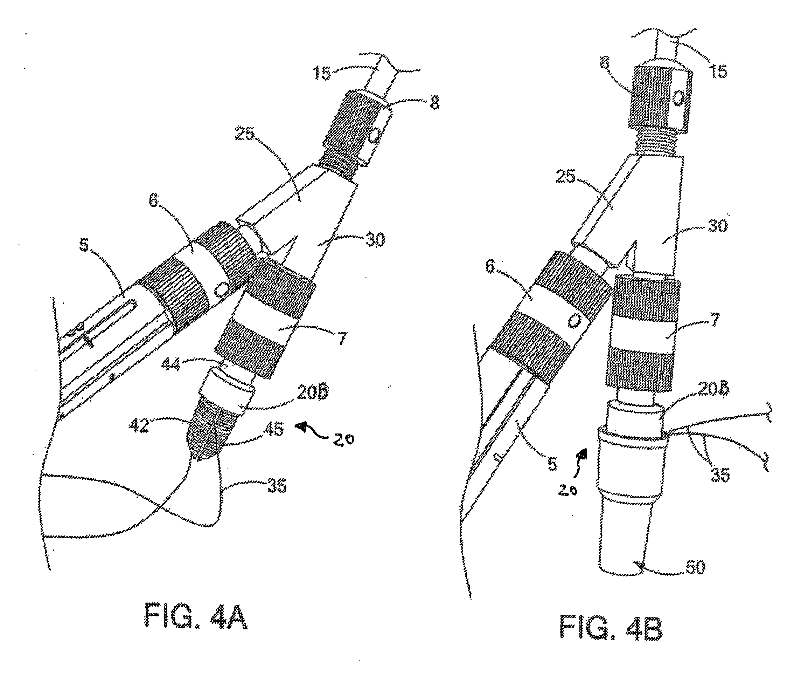

In another embodiment of the present invention, the closure mechanism 20 may be a pin vise collet 20B as shown in Figure 4A. A pin vise collet 20B is a type holding device, similar to a chuck, that includes a slotted cylindrical member or collar 42 having slots 45 that are adjustable in size via a locking cap 50 to exerts a strong clamping force on the suture 35. The collet 20B may have one or more slits 45 in its collar 42. The sutures 35 are fed through the tubular body 44 of the pin vise collet 20B, and then are pulled to one side through one or more of the slits 45 made in the collar 42 of the collet 20B. The appropriate tension to achieve tissue approximation may then be manually obtained by the operator pulling on the sutures 35. Once the desired level of tension is obtained, the tension may be sustained by placing the locking cap 50 onto the collet 20B as shown in Figure 4B. The inner diameter of the locking cap 50 is threaded and sized relative to the outer threads of the collar 42 in order to pinch or otherwise frictionally engage the suture 35. Minute changes in the level of friction can be made by slightly loosening the locking cap 50, and the tension may be adjusted by pulling or releasing the sutures 35 to obtain the desired level of tension and tissue approximation. Retightening the locking cap 50 will then sustain the new level of tension applied to the sutures 35.

The main body 25, side retaining sleeve 30, and closure mechanism 20 of the tension sustaining fixture 10 may be comprised of any known material approved for medical applications. This material may include, but not be limited to stainless steels, titanium, nitinol, ceramic materials, and plastic materials. Examples of plastic materials suitable for this application include polycarbonates, polyamides, polytetrafluorethylenes, polyethylene ether ketones, polyvinylchlorides, polyimides, polyurethanes, and polyethylenes, among others. The sutures 35 may be comprised of any material known to one skilled in the art. Materials, such as 2-0 silik, 2-0 Ti-Cron, 4-0 polypropylene, 5-0 polypropylene, 6-0 polypropylene, and 7-0 polypropylene are preferred.

According to another aspect of the present invention, the medical system useful for suturing tissue in a patient during an intracorporeal procedure basically comprises the combination of the suturing device 2 and the tension sustaining fixture 10, such as those described above. As noted above, the tension sustaining fixture 10 and the suturing device 2 may be integrally and/or unitarily formed as a single unit, or an existing suturing device 2 may be modified to include the tension sustaining fixture 10.

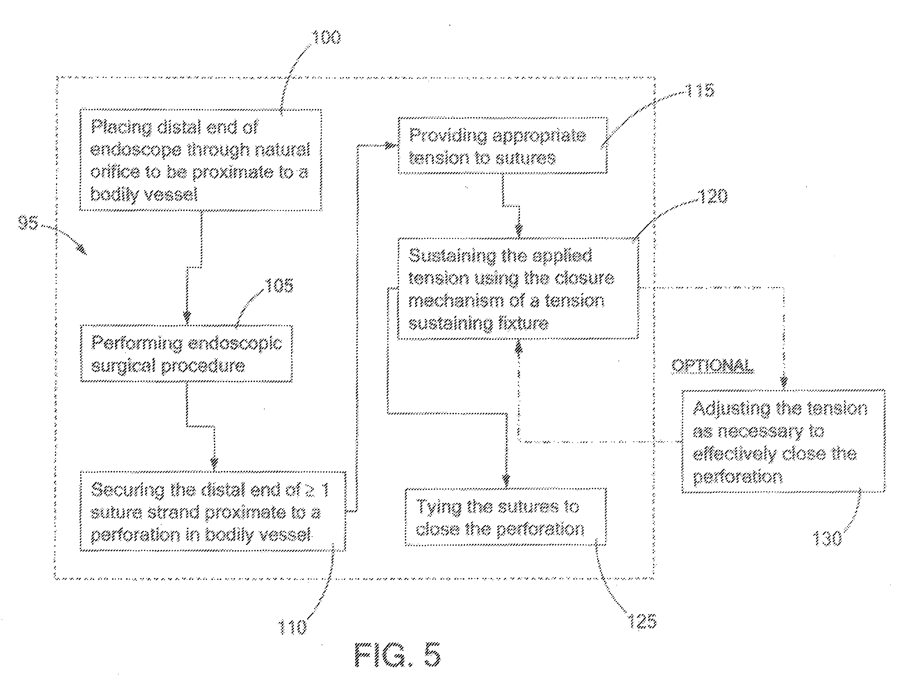

Referring now to Figure 5, an exemplary method 95 for closing perforations made in a material comprises the steps of placing the distal end 100 of the suturing device at a location that is proximate to the material; making a perforation 105 in the material; securing the distal end 110 of at least one suture strand proximate to the perforation made in the material; providing appropriate tension 115 to the sutures; sustaining the applied tension 120 using the tension sustaining fixture; and tying the sutures 125 to close the perforation.

The proximal end of at least one suture is fed from the perforation in the material through the side retaining sleeve and the closure mechanism of the tension sustaining fixture. In the case of an endoscopic or laparoscopic procedure, the proximal end of the suture would be external to the patient. The appropriate tension is then provided and manipulated either manually or through the use of an instrument or a robot.

The applied tension may be sustained by closing the closure mechanism 20 of the tension sustaining fixture 10. Such a closing mechanism may be a rotatable member 20A, such as a stopcock 20A, or a pin vise collet 20B with a locking cap. The method further comprises the step of adjusting 130 the tension as necessary to effectively close the perforation. This type of adjustment may be accomplished by partially opening the closure mechanism.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims.

## Claims

1. A tension sustaining fixture (10) for use with a suturing device (2) during an intracorporeal procedure, the tension sustaining fixture (10) having:
an elongated tube or sheath (15);
a main body (25) having a first passageway (31), the main body (25) capable of being coupled to the elongated sheath (15);
at least one suture strand (35) having a proximal and a distal end;
a closure mechanism (20, 20A, 20B) sized to slidably receive the at least one suture strand (35), the closure mechanism (20, 20A, 20B) being operable between a locked configuration and an unlocked configuration, the locked configuration fixing the at least one suture strand (35) to maintain the tension applied to the at least one suture strand (35);
**characterised in that**
the tension sustaining fixture (10) further comprises a side retaining sleeve (30) coupled to the main body (25), wherein the closure mechanism (20, 20A, 20B) engages the side retaining sleeve (30), and wherein an internal surface of the side retaining sleeve (30) and the closure mechanism (20, 20A, 20B) define a second passageway (32, 40) intersecting with the first passageway (31); and
at least one end of the at least one suture strand (35) extends from the first passageway (31) through the second passageway (32, 40).

2. The tension sustaining fixture (10) of claim 1, wherein the first passageway (31) and the second passageway (32, 40) intersect at an angle (α) having a magnitude less than about 90 degrees.

3. The tension sustaining fixture (10) of claim 1, wherein the amount of tension placed on the suture strand (35) by the closure mechanism (20, 20A, 20B) is negligible when the closure mechanism (20, 20A, 20B) is in an unlocked configuration.

4. The tension sustaining fixture (10) of claim 1, wherein the closure mechanism (20, 20A, 20B) is selected as one from the group of a rotatable member (20A) positioned to close the second passageway (32, 40) and a pin vise collet (20B) having a cap (50) in a tightened or locked position.

5. The tension sustaining fixture (10) of claim 4, wherein the rotatable member (20A) is a stopcock.

6. The tension sustaining fixture (10) of claim 5, wherein the stopcock (20A) comprises a rotatable key (22), a fixed body (21) and a handle (23).

7. The tension sustaining fixture (10) of claim 5, wherein the fixture is configured such that minute adjustments to the amount of tension applied to the suture strand (35) is accomplished by rotating the stopcock (20A) to a partially open or closed position.

8. The tension sustaining fixture (10) of claim 4, wherein the pin vise collet (20B) comprises at least one slit (45) in its body (44) or collar (42) that engages the tensioned suture strand (35) when the cap (50) is tightened.

9. The tension sustaining fixture (10) of claim 8, wherein the fixture is configured such that minute adjustments to the amount of tension applied to the suture strand (35) is accomplished by loosening the locking cap (50) and manually adjusting the tension applied.

10. The tension sustaining fixture (10) of claim 1, wherein the intracorporeal procedure is an endoscopic procedure using an endoscope and wherein the elongated tube or sheath (15) of the tension sustaining fixture (10) is sized to be inserted through a working channel of the endoscope.

11. A medical system (1) for suturing tissue in an intracorporeal procedure in a patient, the medical system 1 comprising the tension sustaining fixture (10) according to any of claims 1 to 10 and further comprising:
a suturing device (2) having a handle (5) and an outer sheath that slidably receives the distal end of the suture strands (35) for delivery to the tissue within a patient;
wherein the main body (25) of the tension sustaining fixture (10) is located between the handle (5) and the outer sheath of the suturing device (2); and
wherein a proximal end of the at least one suture strand (35) follows a path from the outer sheath of the suturing device (2) through the tension sustaining fixture (10) to be external to the patient.

## Patentansprüche

1. Spannungsaufrechterhaltungsvorrichtung (10) zur Verwendung mit einer Nähvorrichtung (2) während eines intrakorporalen Verfahrens, wobei die Spannungsaufrechterhaltungsvorrichtung (10) umfasst:
einen länglichen Schlauch oder Mantel (15);
einen Hauptkörper (25), der einen ersten Durchgang (31) aufweist, wobei der Hauptkörper (25) dazu geeignet ist, mit dem länglichen Mantel (15) verbunden zu werden;
mindestens einen Fadenstrang (35), der ein proximales und ein distales Ende aufweist;
einen Verschließmechanismus (20, 20A, 20B), der derart dimensioniert ist, um den mindestens einen Fadenstrang (35) verschiebbar aufzunehmen, wobei der Verschließmechanismus (20, 20A, 20B) zwischen einer verriegelten Konfiguration und einer entriegelten Konfiguration betätigbar ist, wobei die verriegelte Konfiguration den mindestens einen Fadenstrang (35) fixiert, um die Spannung aufrecht zu erhalten, die auf den mindestens einen Fadenstrang (35) aufgebracht wird;
**dadurch gekennzeichnet, dass**
die Spannungsaufrechterhaltungsvorrichtung (10) weiterhin eine seitliche Haltehülse (30) umfasst, die mit dem Hauptkörper (25) verbunden ist, wobei der Verschließmechanismus (20, 20A, 20B) mit der seitlichen Haltehülse (30) in Eingriff steht, und wobei eine innere Oberfläche der seitlichen Haltehülse (30) und des Verschließmechanismus (20, 20A, 20B) einen zweiten Durchgang (32, 40) definieren, der sich mit dem ersten Durchgang (31) schneidet; und
sich mindestens ein Ende des mindestens einen Fadenstrangs (35) von dem ersten Durchgang (31) durch den zweiten Durchgang (32, 40) hindurch erstreckt.

2. Spannungsaufrechterhaltungsvorrichtung (10) nach Anspruch 1, wobei sich der erste Durchgang (31) und der zweite Durchgang (32, 40) in einem Winkel (α) schneiden, der kleiner als in etwa 90° ist.

3. Spannungsaufrechterhaltungsvorrichtung (10) nach Anspruch 1, wobei die Höhe der Spannung, die durch den Verschließmechanismus (20, 20A, 20B) auf den Fadenstrang (35) aufgebracht wird, vernachlässigbar ist, wenn der Verschließmechanismus (20, 20A, 20B) in einer entriegelten Konfiguration ist.

4. Spannungsaufrechterhaltungsvorrichtung (10) nach Anspruch 1, wobei der Verschließmechanismus (20, 20A, 20B) ausgewählt ist aus der Gruppe bestehend aus einem drehbaren Element (20A), das derart angeordnet ist, um den zweiten Durchgang (32, 40) zu schließen, und einer Einspann-Klemmhülse (20B), die einen Aufsatz in einer angezogenen oder verriegelten Position aufweist.

5. Spannungsaufrechterhaltungsvorrichtung (10) nach Anspruch 4, wobei das drehbare Element (20A) ein Absperrorgan ist.

6. Spannungsaufrechterhaltungsvorrichtung (10) nach Anspruch 5, wobei das Absperrorgan (20A) einen drehbaren Schlüssel (22), einen feststehenden Körper (21) und einen Griff (23) umfasst.

7. Spannungsaufrechterhaltungsvorrichtung (10) nach Anspruch 5, wobei die Vorrichtung derart ausgestaltet ist, so dass exakte Anpassungen der Höhe der Spannung, die auf den Fadenstrang (35) aufgebracht wird, durch Drehen des Absperrorgans (20A) in eine teilweise geöffnete oder geschlossene Position erreicht werden können.

8. Spannungsaufrechterhaltungsvorrichtung (10) nach Anspruch 4, wobei die Einspann-Klemmhülse (20B) mindestens einen Schlitz (45) in ihrem Körper (44) oder Kranz (42) umfasst, der den gespannten Fadenstrang (35) in Eingriff nimmt, wenn der Aufsatz (50) angezogen wird.

9. Spannungsaufrechterhaltungsvorrichtung (10) nach Anspruch 8, wobei die Vorrichtung derart ausgestaltet ist, dass exakte Anpassungen der Höhe der Spannung, die auf den Fadenstrang (35) aufgebracht wird, durch Lockern des Verschlussaufsatzes (50) und durch manuelles Anpassen der aufgebrachten Spannung erreicht werden können.

10. Spannungsaufrechterhaltungsvorrichtung (10) nach Anspruch 1, wobei das intrakorporale Verfahren ein endoskopisches Verfahren ist, das ein Endoskop verwendet, und wobei der längliche Schlauch oder Mantel (15) der Spannungsaufrechterhaltungsvorrichtung (10) derart dimensioniert ist, dass er durch einen Arbeitskanal des Endoskops eingeführt werden kann.

11. Medizinisches System (1) zum Nähen von Gewebe in einem intrakorporalen Verfahren in einem Patienten, wobei das medizinische System (1) die Spannungsaufrechterhaltungsvorrichtung (10) nach einem der Ansprüche 1 bis 10 aufweist und weiterhin umfasst:
eine Nähvorrichtung (2), die einen Griff (5) und einen äußeren Mantel umfasst, der das distale Ende der Fadenstränge (35) verschiebbar aufnimmt, um es zu dem Gewebe innerhalb eines Patienten zu transportieren;
wobei der Hauptkörper (25) der Spannungsaufrechterhaltungsvorrichtung (10) zwischen dem Griff (5) und dem äußeren Mantel der Nähvorrichtung (2) angeordnet ist; und
wobei ein proximales Ende des mindestens einen Fadenstrangs (35) einem Pfad von dem äußeren Mantel der Nähvorrichtung (2) durch die Spannungsaufrechterhaltungsvorrichtung (10) folgt, um außerhalb des Patienten zu sein.

## Revendications

1. Fixation (10) pour maintenir la tension destinée à être utilisée avec un dispositif (2) de suture au cours d'une procédure intracorporelle, la fixation (10) pour maintenir la tension possédant :
un tube allongé ou gaine (15) ;
un corps (25) principal présentant un premier passage (31), le corps (25) principal pouvant être accouplé à la gaine (15) allongée ;
au moins un fil (35) de suture présentant une extrémité proximale et une extrémité distale ;
un mécanisme (20, 20A, 20B) de fermeture dimensionné pour recevoir par coulissement ledit au moins fil (35) de suture, le mécanisme (20, 20A, 20B) de fermeture pouvant être commandé entre une configuration verrouillée et une configuration déverrouillée, la configuration verrouillée fixant ledit au moins un fil (35) de suture pour maintenir la tension appliquée sur ledit au moins un fil (35) de suture ;
**caractérisée en ce que**
la fixation (10) pour maintenir la tension comporte en outre un manchon (30) de retenue latéral accouplé au corps (25) principal, **en ce que** le mécanisme (20, 20A, 20B) de fermeture se met en prise avec le manchon (30) de retenue latéral, et **en ce qu'**une surface interne du manchon (30) de retenue latéral et le mécanisme (20, 20A, 20B) de fermeture délimitent un deuxième passage (32, 40) coupant le premier passage (31) ; et
au moins une extrémité dudit au moins un fil (35) de suture s'étend de part en part du premier passage (31) au deuxième passage (32, 40).

2. Fixation (10) pour maintenir la tension selon la revendication 1, dans lequel le premier passage (31) et le deuxième passage (32, 40) se coupent à un angle (α) dont l'ouverture est inférieure à environ 90 degrés.

3. Fixation (10) pour maintenir la tension selon la revendication 1, dans laquelle la quantité de tension placée sur le fil (35) de suture par le mécanisme (20, 20A, 20B) de fermeture est négligeable lorsque le mécanisme (20, 20A, 20B) de fermeture est dans une configuration déverrouillée.

4. Fixation (10) pour maintenir la tension selon la revendication 1, dans laquelle le mécanisme (20, 20A, 20B) de fermeture est sélectionné dans le groupe constitué d'un élément (20A) rotatif positionné pour fermer le deuxième passage (32, 40) et/ou une pince (20B) à serrage par étau munie d'un capuchon (50) dans une position serrée ou verrouillée.

5. Fixation (10) pour maintenir la tension selon la revendication 4, dans laquelle l'élément (20A) rotatif est un robinet d'arrêt.

6. Fixation (10) pour maintenir la tension selon la revendication 5, dans laquelle le robinet d'arrêt (20A) comporte une clé (22) rotative, un corps (21) fixé et un manche (23).

7. Fixation (10) pour maintenir la tension selon la revendication 5, dans laquelle la fixation est configurée de sorte que de minuscules réglages de la quantité de tension appliquée sur le fil (35) de suture sont réalisés en faisant tourner le robinet d'arrêt (20A) a une position partiellement ouverte ou fermée.

8. Fixation (10) pour maintenir la tension selon la revendication 4, dans laquelle la pince (20B) à serrage par étau comporte au moins une fente (45) dans son corps (44) ou une douille (42) qui se met en prise avec le fil (35) de suture sous tension lorsque le capuchon (50) est serré.

9. Fixation (10) pour maintenir la tension selon la revendication 8, dans laquelle la fixation est configurée de sorte que de minuscules réglages de la quantité de tension appliquée sur le fil (35) de suture sont réalisés en desserrant le capuchon (50) de verrouillage et en réglant manuellement la tension appliquée.

10. Fixation (10) pour maintenir la tension selon la revendication 1, dans laquelle la procédure intracorporelle est une procédure endoscopique qui utilise un endoscope et dans laquelle le tube allongé ou gaine (15) de la fixation (10) pour maintenir la tension est dimensionnée de manière à être introduite dans un canal de travail de l'endoscope.

11. Système (1) médical destiné à suturer des tissus au cours d'une procédure intracorporelle sur un patient, le système (1) médical comportant la fixation (10) pour maintenir la tension selon l'une quelconque des revendications 1 à 10 et comportant en outre :
un dispositif (2) de suture muni d'une poignée (5) et d'une gaine externe qui reçoit par coulissement l'extrémité distale des fils (35) de suture afin de les mettre en place dans les tissus à l'intérieur d'un patient ;
dans lequel le corps (25) principal de la fixation (10) pour maintenir la tension est situé entre la poignée (5) et la gaine externe du dispositif (2) de suture ; et
dans lequel une extrémité proximale dudit au moins un fil (35) de suture suit un trajet depuis la gaine externe du dispositif (2) de suture à travers la fixation (10) pour maintenir la tension afin d'être à l'extérieur du patient.
